# EUROPEAN PATENT SPECIFICATION

(11) **EP 0 868 197 B1**
(45) Date of publication and mention of the grant of the patent: **06.05.2004**
(21) Application number: 96928160.9
(22) Date of filing: 14.08.1996
(51) Int. Cl.: A61K 39/395

(54) **ANTI-L-SELECTIN ANTIBODIES FOR PREVENTION OF MULTIPLE ORGAN FAILURE AND ACUTE ORGAN DAMAGE**
ANTIKÖRPER GEGEN L-SELECTIN ZUR VORBEUGUNG VON MULTIPLEM ORGANVERSAGEN UND AKUTER ORGANSCHÄDIGUNG
ANTICORPS ANTI-L-SELECTINE SERVANT A PREVENIR DES DEFAILLANCES ORGANIQUES MULTIPLES ET DES LESIONS ORGANIQUES AIGUES

(30) Priority: 17.08.1995 EP 95112895; 19.09.1995 EP 95114696; 27.12.1995 US 578953
(43) Date of publication of application: 07.10.1998
(73) Proprietor: PROTEIN DESIGN LABS, INC., Fremont, CA 94555 (US)
(72) Inventor: HASELBECK, Anton, D-82362 Weilheim (DE); SCHUMACHER, Günther, D-82347 Benried (DE); CO, Man, Sung, Cupertino, CA 95014 (US); MARTIN, Ulrich, D-80469 München (DE)
(74) Representative: Huber, Bernhard, Dipl.-Chem.
(86) International application number: PCT/US1996/013152
(87) International publication number: WO 1997/006822

(56) References cited:
- WO-A-93/00111
- WO-A-93/02698
- WO-A-94/12215
- WO-A-95/15181
- N.E. MOAT ET AL.: "HUMORAL AND CELLULAR ACTIVATION IN A SIMULATED EXTRACORPOREAL CIRCUIT." THE ANNALS OF THORACIC SURGERY, vol. 56, no. 6, pages 1509-1514, XP000562807 AMSTERDAM, NL
- A. FINN ET AL.: "SYSTEMIC INFLAMMATION DURING PAEDIATRIC CARDIOPULMONARY BYPASS: CHANGES IN NEUTROPHIL ADHESIVE PROPERTIES." PERFUSION, vol. 8, no. 1, pages 39-48, XP000563713
- J. PHARMACOL. EXP. THERAP., Vol. 271, Number 1, issued 144, BUERKE et al., "Humanized Monoclonal Antibody DREG-200 Directed Against L-Selectin Protects in Feline Myocardial Reperfusion Injury", pages 134-142.
- BEHRING INST. MITT., Volume 92, issued 1993, WINN et al., "Monoclonal Antibodies to Leukocyte and Endothelial Adhesion Molecules Attentuate Ischemia-Reperfusion Injury", pages 229-237.
- ANN. THORAC. SURG., Volume 56, issued 1993, ELLIOTT et al., "Interaction Between Neutrophils and Endothelium", pages 1503-1508.
- BLOOD, Volume 79, Number 5, issued 01 March 1992, RINDER et al., "Cardipulmonary Bypass Induces Leukocyte-Platelet Adhesion", pages 1201-1205.
- STÜRM ET AL: 'Cardiopulmonary Parameters and Prognosis after Severe Multiple Trauma' JOURNAL OF TRAUMA vol. 19, no. 5, May 1979, pages 305 - 318
- NAST-KOLB ET AL: 'Biochemische Faktoren als objektive Parameter zur Prognoseabschätzung beim Polytrauma' UNFALLCHIRURG vol. 95, 1992, pages 59 - 66
- REGEL ET AL: 'Ergebnisse in der Behandlung Poytraumatisierter' UNFALLCHIRURG vol. 96, 1993, pages 350 - 362
- LEHMANN ET AL: 'Hat die Initialversorgung des polytraumatisierten Patienten Einfluss auf die Ausbildung eines multiplen Organversagens?' UNFALLCHIRURG vol. 98, 1995, pages 442 - 446
- CIVL ET AL: 'The Abbreviated Injury Scale, 1985 Revision: A Condensed Chart For Clinical Use' JOURNAL OF TRAUMA vol. 28, no. 1, January 1988, pages 87 - 90
- HARLAN & WINN: 'Leukocyte-endothelial interactions: Clinical trials of anti-adhesion therapy' CRIT CARE MED vol. 30, no. 5, 2002, pages S214 - S219

### SEQUENCE LISTING

(1) GENERAL INFORMATION:
   (i) APPLICANTS:
      (A) NAME: Martin, Ulrich, et al.
   (ii) TITLE OF INVENTION: Anti-L-selectin antibodies for prevention of multiple organ failure after polytrauma and for prevention of acute organ damage after extracorporeal blood circulation.
   (iii) NUMBER OF SEQUENCES: 4
   (iv) CORRESPONDENCE ADDRESS:
      (A) Felfe & Lynch
         Attn: Norman D. Hanson
      (B) 805 Third Avenue
      (C) New York
      (D) New York
      (E) U.S.A.
      (F) 10022
   (v) COMPUTER READABLE FORM:
      (A) MEDIUM TYPE: 3.5" Computer Disk
      (B) COMPUTER: IBM PC compatible
      (C) OPERATING SYSTEM: PC-DOS/MS-DOS
      (D) SOFTWARE: ASCII
   (vi) CURRENT APPLICATION DATA:
      (A) APPLICATION NUMBER: To be assigned
      (B) 20-Dec-95
      (C) CLASSIFICATION: 530
   (vii) PRIOR APPLICATION DATA:
      (A) APPLICATION NUMBER: EP 95 112 895.8
      (B) FILING DATE: 17-Aug-1995
      (A) APPLICATION NUMBER: EP 95 114 969.9
      (B) FILING DATE: 19-Sep-1995
      (A) APPLICATION NUMBER: US 08 578 953
      (B) FILING DATE: 27-Dec-1995
   (viii) ATTORNEY/AGENT INFORMATION
      (A) NAME: Hanson, Norman D.
      (B) REGISTRATION NUMBER: 30,946
      (C) REFERENCE/DOCKET NUMBER: BOER 1059-PFF/NDH
   (ix) TELECOMMUNICATION INFORMATION
      (A) TELEPHONE: (212) 688-9200
      (B) TELEFAX: (212) 838-3884
(2) INFORMATION FOR SEQ ID NO: 1:
   (i) SEQUENCE CHARACTERISTICS:
      (A) LENGTH: 654 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION: 1,,654
   (xi) SEQUENCE DESCRIPTION: SEQ ID NO: 1:
   GAC ATT CAG ATG ACC CAA TCT CCG AGC TCT TTG TCT GCG TCT GTA GGG  48
   Asp Ile Gln Met Thr Gln Ser Pro Ser Ser Leu Ser Ala Ser Val Gly
   1               5                   10                  15
   GAT AGG GTC ACT ATC ACC TGC AAG GCC AGC CAA AGT GTT GAT TAT GAT  96
   Asp Arg Val Thr Ile Thr Cys Lys Ala Ser Gln Ser Val Asp Tyr Asp
               20                  25                  30
   GGT GAT AGT TAT ATG AAC TGG TAC CAA CAG AAA CCA GGA AAG GCA CCC 144
   Gly Asp Ser Tyr Met Asn Trp Tyr Gln Gln Lys Pro Gly Lys Ala Pro
           35                  40                  45
   AAG CTT CTC ATC TAT GCT GCA TCC AAC CTA GAA TCT GGT ATC CCA TCC 192
   Lys Leu Leu Ile Tyr Ala Ala Ser Asn Leu Glu Ser Gly Ile Pro Ser
       50                  55                  60
   AGG TTT AGT GGC AGT GGG TCT GGG ACA GAC TTC ACC CTC ACC ATC TCT 240
   Arg Phe Ser Gly Ser Gly Ser Gly Thr Asp Phe Thr Leu Thr Ile Ser
   65                  70                  75                  80
   TCT CTG CAG CCG GAG GAT TTC GCA ACC TAT TAC TGT CAG CAA AGT AAT 288
   Ser Leu Gln Pro Glu Asp Phe Ala Thr Tyr Tyr Cys Gln Gln Ser Asn
                   85                  90                  95
   GAA GAT CCG TGG ACG TTC GGT CAA GGC ACC AAG GTG GAA ATC AAA CGA 336
   Glu Asp Pro Trp Thr Phe Gly Gln Gly Thr Lys Val Glu Ile Lys Arg
               100                 105                 110
   ACT GTG GCT GCA CCA TCT GTC TTC ATC TTC CCG CCA TCT GAT GAG CAG 384
   Thr Val Ala Ala Pro Ser Val Phe Ile Phe Pro Pro Ser Asp Glu Gln
           115                 120                 125
   TTG AAA TCT GGA ACT GCC TCT GTT GTG TGC CTG CTG AAT AAC TTC TAT 432
   Leu Lys Ser Gly Thr Ala Ser Val Val Cys Leu Leu Asn Asn Phe Tyr
       130                 135                 140
   CCC AGA GAG GCC AAA GTA CAG TGG AAG GTG GAT AAC GCC CTC CAA TCG 480
   Pro Arg Glu Ala Lys Val Gln Trp Lys Val Asp Asn Ala Leu Gln Ser
   145                 150                 155                 160
   GGT AAC TCC CAG GAG AGT GTC ACA GAG CAG GAC AGC AAG GAC AGC ACC 528
   Gly Asn Ser Gln Glu Ser Val Thr Glu Gln Asp Ser Lys Asp Ser Thr
                   165                 170                 175
   TAC AGC CTC AGC AGC ACC CTG ACG CTG AGC AAA GCA GAC TAC GAG AAA 576
   Tyr Ser Leu Ser Ser Thr Leu Thr Leu Ser Lys Ala Asp Tyr Glu Lys
               180                 185                 190
   CAC AAA GTC TAC GCC TGC GAA GTC ACC CAT CAG GGC CTG AGC TCG CCC 624
   His Lys Val Tyr Ala Cys Glu Val Thr His Gln Gly Leu Ser Ser Pro
           195                 200                 205
   GTC ACA AAG AGC TTC AAC AGG GGA GAG TGT                         654
   Val Thr Lys Ser Phe Asn Arg Gly Glu Cys
       210                 215
(2) INFORMATION FOR SEQ ID NO: 2:
   (i) SEQUENCE CHARACTERISTICS:
      (A) LENGTH: 218
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 2:
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Ser Val Asp Tyr Asp
               20                  25                  30
   Gly Asp Ser Tyr Met Asn Trp Tyr Gln Gln Lys Pro Gly Lys Ala Pro
           35                  40                  45
   Lys Leu Leu Ile Tyr Ala Ala Ser Asn Leu Glu Ser Gly Ile Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln Gln Ser Asn
                   85                  90                  95
   Glu Asp Pro Trp Thr Phe Gly Gln Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
(2) INFORMATION FOR SEQ ID NO: 3:
   (i) SEQUENCE CHARACTERISTICS:
      (A) LENGTH: 1329 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION:1,,1329
   (ix) FEATURE:
      (A) NAME/KEY: mat_peptide
      (B) LOCATION:1
   (xi) SEQUENCE DESCRIPTION: SEQ ID NO: 3:
   GAA GTG CAA CTG GTG GAG TCT GGG GGA GGC TTA GTG CAG CCT GGA GGA   48
   Glu Val Gln Leu Val Glu Ser Gly Gly Gly Leu Val Gln Pro Gly Gly
   1               5                   10                  15
   AGC TTG AGA CTC TCC TGT GCA GCC TCT GGA TTC ACT TTC AGT ACC TAT   96
   Ser Leu Arg Leu Ser Cys Ala Ala Ser Gly Phe Thr Phe Ser Thr Tyr
               20                  25                  30
   GCC ATG TCT TGG GTT CGC CAG GCT CCA GGG AAG GGA CTC GAG TGG GTC  144
   Ala Met Ser Trp Val Arg Gln Ala Pro Gly Lys Gly Leu Glu Trp Val
           35                  40                  45
   GCA TCC ATT AGT ACT GGT GGT AGC ACC TAC TAT CCA GAC AGT GTG AAG 192
   Ala Ser Ile Ser Thr Gly Gly Ser Thr Tyr Tyr Pro Asp Ser Val Lys
       50                  55                  60
   GGC CGA TTC ACC ATC TCC AGA GAT AAT GCC AAG AAC ACC CTG TAC CTG 240
   Gly Arg Phe Thr Ile Ser Arg Asp Asn Ala Lys Asn Thr Leu Tyr Leu
   65                  70                  75                  80
   CAA ATG AAT TCT CTG AGG GCT GAG GAC ACG GCC GTG TAT TAC TGT GCA 288
   Gln Met Asn Ser Leu Arg Ala Glu Asp Thr Ala Val Tyr Tyr Cys Ala
                   85                  90                  95
   AGA GAC TAT GAC GGG TAT TTT GAC TAC TGG GGC CAA GGC ACC CTG GTC 336
   Arg Asp Tyr Asp Gly Tyr Phe Asp Tyr Trp Gly Gln Gly Thr Leu Val
               100                 105                 110
   ACA GTC TCC TCA GCT TCC ACC AAG GGC CCA TCC GTC TTC CCC CTG GCG 384
   Thr Val Ser Ser Ala Ser Thr Lys Gly Pro Ser Val Phe Pro Leu Ala
           115                 120                 125
   CCC TGC TCC AGG AGC ACC TCC GAG AGC ACA GCC GCC CTG GGC TGC CTG 432
   Pro Cys Ser Arg Ser Thr Ser Glu Ser Thr Ala Ala Leu Gly Cys Leu
       130                 135                 140
   GTC AAG GAC TAC TTC CCC GAA CCG GTG ACG GTG TCG TGG AAC TCA GGC 480
   Val Lys Asp Tyr Phe Pro Glu Pro Val Thr Val Ser Trp Asn Ser Gly
   145                 150                 155                 160
   GCC CTG ACC AGC GGC GTG CAC ACC TTC CCG GCT GTC CTA CAG TCC TCA 528
   Ala Leu Thr Ser Gly Val His Thr Phe Pro Ala Val Leu Gln Ser Ser
                   165                 170                 175
   GGA CTC TAC TCC CTC AGC AGC GTG GTG ACC GTG CCC TCC AGC AGC TTG 576
   Gly Leu Tyr Ser Leu Ser Ser Val Val Thr Val Pro Ser Ser Ser Leu
               180                 185                 190
   GGC ACG AAG ACC TAC ACC TGC AAC GTA GAT CAC AAG CCC AGC AAC ACC 624
   Gly Thr Lys Thr Tyr Thr Cys Asn Val Asp His Lys Pro Ser Asn Thr
           195                 200                 205
   AAG GTG GAC AAG AGA GTT GAG TCC AAA TAT GGT CCC CCA TGC CCA TCA 672
   Lys Val Asp Lys Arg Val Glu Ser Lys Tyr Gly Pro Pro Cys Pro Ser
       210                 215                 220
   TGC CCA GCA CCT GAG TTC CTG GGG GGA CCA TCA GTC TTC CTG TTC CCC 720
   Cys Pro Ala Pro Glu Phe Leu Gly Gly Pro Ser Val Phe Leu Phe Pro
   225                 230                 235                 240
   CCA AAA CCC AAG GAC ACT CTC ATG ATC TCC CGG ACC CCT GAG GTC ACG 768
   Pro Lys Pro Lys Asp Thr Leu Met Ile Ser Arg Thr Pro Glu Val Thr
                   245                 250                 255
   TGC GTG GTG GTG GAC GTG AGC CAG GAA GAC CCC GAG GTC CAG TTC AAC 816
   Cys Val Val Val Asp Val Ser Gln Glu Asp Pro Glu Val Gln Phe Asn
               260                 265                 270
   TGG TAC GTG GAT GGC GTG GAG GTG CAT AAT GCC AAG ACA AAG CCG CGG 864
   Trp Tyr Val Asp Gly Val Glu Val His Asn Ala Lys Thr Lys Pro Arg
           275                 280                 285
   GAG GAG CAG TTC AAC AGC ACG TAC CGT GTG GTC AGC GTC CTC ACC GTC 912
   Glu Glu Gln Phe Asn Ser Thr Tyr Arg Val Val Ser Val Leu Thr Val
       290                 295                 300
   CTG CAC CAG GAC TGG CTG AAC GGC AAG GAG TAC AAG TGC AAG GTC TCC 960
   Leu His Gln Asp Trp Leu Asn Gly Lys Glu Tyr Lys Cys Lys Val Ser
   305                 310                 315                 320
   AAC AAA GGC CTC CCG TCC TCC ATC GAG AAA ACC ATC TCC AAA GCC AAA 1008
   Asn Lys Gly Leu Pro Ser Ser Ile Glu Lys Thr Ile Ser Lys Ala Lys
                   325                 330                 335
   GGG CAG CCC CGA GAG CCA CAG GTG TAC ACC CTG CCC CCA TCC CAG GAG 1056
   Gly Gln Pro Arg Glu Pro Gln Val Tyr Thr Leu Pro Pro Ser Gln Glu
               340                 345                 350
   GAG ATG ACC AAG AAC CAG GTC AGC CTG ACC TGC CTG GTC AAA GGC TTC 1104
   Glu Met Thr Lys Asn Gln Val Ser Leu Thr Cys Leu Val Lys Gly Phe
           355                 360                 365
   TAC CCC AGC GAC ATC GCC GTG GAG TGG GAG AGC AAT GGG CAG CCG GAG 1152
   Tyr Pro Ser Asp Ile Ala Val Glu Trp Glu Ser Asn Gly Gln Pro Glu
       370                 375                 380
   AAC AAC TAC AAG ACC ACG CCT CCC GTG CTG GAC TCC GAC GGC TCC TTC 1200
   Asn Asn Tyr Lys Thr Thr Pro Pro Val Leu Asp Ser Asp Gly Ser Phe
   385                 390                 395                 400
   TTC CTC TAC AGC AGG CTA ACC GTG GAC AAG AGC AGG TGG CAG GAG GGG 1248
   Phe Leu Tyr Ser Arg Leu Thr Val Asp Lys Ser Arg Trp Gln Glu Gly
                   405                 410                 415
   AAT GTC TTC TCA TGC TCC GTG ATG CAT GAG GCT CTG CAC AAC CAC TAC 1296
   Asn Val Phe Ser Cys Ser Val Met His Glu Ala Leu His Asn His Tyr
               420                 425                 430
   ACA CAG AAG AGC CTC TCC CTG TCT CTG GGT AAA                     1329
   Thr Gln Lys Ser Leu Ser Leu Ser Leu Gly Lys
           435                 440
(2) INFORMATION FOR SEQ ID NO: 4:
   (i) SEQUENCE CHARACTERISTICS:
      (A) LENGTH: 443
      (B) TYPE: amino acid
      (C) STRANDEDNESS: double
      (D) TOPOLOGY: linear
   (ii) MOLECULE TYPE: protein
   (xi) SEQUENCE DESCRIPTION: SEQ ID NO: 4:
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Ala Met Ser Trp Val Arg Gln Ala Pro Gly Lys Gly Leu Glu Trp Val
           35                  40                  45
   Ala Ser Ile Ser Thr Gly Gly Ser Thr Tyr Tyr Pro Asp Ser Val Lys
       50                  55                  60
   Gly Arg Phe Thr Ile Ser Arg Asp Asn Ala Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Tyr Asp Gly Tyr Phe Asp Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Cys Ser Arg Ser Thr Ser Glu Ser Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Lys Thr Tyr Thr Cys Asn Val Asp His Lys Pro Ser Asn Thr
           195                 200                 205
   Lys Val Asp Lys Arg Val Glu Ser Lys Tyr Gly Pro Pro Cys Pro Ser
       210                 215                 220
   Cys Pro Ala Pro Glu Phe Leu Gly Gly Pro Ser Val Phe Leu Phe Pro
   225                 230                 235                 240
   Pro Lys Pro Lys Asp Thr Leu Met Ile Ser Arg Thr Pro Glu Val Thr
                   245                 250                 255
   Cys Val Val Val Asp Val Ser Gln Glu Asp Pro Glu Val Gln Phe Asn
               260                 265                 270
   Trp Tyr Val Asp Gly Val Glu Val His Asn Ala Lys Thr Lys Pro Arg
           275                 280                 285
   Glu Glu Gln Phe Asn Ser Thr Tyr Arg Val Val Ser Val Leu Thr Val
       290                 295                 300
   Leu His Gln Asp Trp Leu Asn Gly Lys Glu Tyr Lys Cys Lys Val Ser
   305                 310                 315                 320
   Asn Lys Gly Leu Pro Ser Ser Ile Glu Lys Thr Ile Ser Lys Ala Lys
                   325                 330                 335
   Gly Gln Pro Arg Glu Pro Gln Val Tyr Thr Leu Pro Pro Ser Gln Glu
               340                 345                 350
   Glu Met Thr Lys Asn Gln Val Ser Leu Thr Cys Leu Val Lys Gly Phe
           355                 360                 365
   Tyr Pro Ser Asp Ile Ala Val Glu Trp Glu Ser Asn Gly Gln Pro Glu
       370                 375                 380
   Asn Asn Tyr Lys Thr Thr Pro Pro Val Leu Asp Ser Asp Gly Ser Phe
   385                 390                 395                 400
   Phe Leu Tyr Ser Arg Leu Thr Val Asp Lys Ser Arg Trp Gln Glu Gly
                   405                 410                 415
   Asn Val Phe Ser Cys Ser Val Met His Glu Ala Leu His Asn His Tyr
               420                 425                 430
   Thr Gln Lys Ser Leu Ser Leu Ser Leu Gly Lys
           435                 440

## Description

### FIELD OF THE INVENTION

This invention relates to the use of anti-L-selectin antibodies for the prevention of multiple organ failure associated with polytrauma.

### BACKGROUND OF THE INVENTION

A polytrauma is understood as an injury of a number of tissues (bones or soft tissues). In a polytraumatic event, mediator systems (e.g. cytokines, arachidonic acid products, oxygen radicals, proteases) as well as leukocytes and other cells are activated. This can lead to secondary organ damage (e.g. destruction of tissue structures by liberated proteases). This secondary organ damage can occur in the whole body independently of the site of the primary trauma.

A polytrauma may also be associated with hemorrhagic shock. Hemorrhagic shock is understood as a shock which is characterized by a rapid and substantial loss of blood toward the inside or outside. At present, hemorrhagic shock can be treated successfully by intensive medical therapy, especially by volume substitution and blood transfusion. The combination of hemorrhagic shock and trauma is referred to as hemorrhagic-traumatic shock. In contrast to pure hemorrhagic shock, there is, at present, no specific therapy for traumatic or hemorrhagic-traumatic shock and no prophylaxis at all for later organ failure following polytrauma.

Multiple organ failure (MOF) is a severe problem which often occurs after polytraumas. The more organs affected, the higher the mortality. Organs and systems which can fail include the heart, lung, kidney, liver, stomach, intestinal system and central nervous system. Although in recent years it has been possible to reduce the very high mortality of trauma patients to about 20 % by improvements in rescue service and emergency medicine, so far there is no specific therapy for organ failure.

Marzi et al., J. Trauma 35: 110-119 (1993) discloses that superoxide dimutase can be given 24 hours after trauma. However, the results are not unequivocal and merely show a trend towards a partial improvement. A substantial reduction in mortality and MOP was, however, not observed. Mileski, W.J. et al., Surgery 108: 206-212 (1990) discloses that the binding of neutrophils or their aggregation contributes substantially to the development of hemorrhagic shock after organ damage. In this case, experimental animals were given anti-CD18 antibodies immediately after a 90 minute phase of hemorrhagic shock. Therapeutic methods for treatment of multiple organ failure after polytrauma is not described by Mileski. Vedder N.B. et al., Surgery 106: 509-516 (1989) also propose the use of anti-CD18 antibodies to treat hemorrhagic shock.

Selectins, such as L, E, and P-selectin, have been found to be associated with tissue damage during the course of ischemia and reperfusion. Neutrophils play an important role in this connection. It is assumed that selectin is required for the recruitment of neutrophils. Apparently L-selectin is necessary for the complete development of damage in skeletal muscle as well as in the lung (Seekamp A. et al., Am. J. Pathol. 11: 592-598 (1994). Mulligan, M.S. et al., J. Immunol. 151: 832-840 (1994) describe a similar phenomenon.

The production of humanized anti-L-selectin antibodies is described in WO 94/12215.
The use of such antibodies in the treatment of inflammatory diseases and in particular of myocardial infarction is proposed. A dose of 1 - 50 mg is proposed to prevent acute lung failure. However, the reference does not describe a method for preventing MOF after polytrauma.

Thus, there is a need for effective treatment of preventing and/or treating multiple organ failure after polytrauma.

### OBJECTS OF THE INVENTION

An object of the invention is to provide a method and a therapeutic composition which can be used to effectively prevent multiple organ failure after polytrauma in humans and to considerably reduce the mortality rate of polytrauma patients. The invention concerns the use of anti-L-selectin antibodies therapeutically and for the production of pharmaceutical compositions useful in the prevention of multiple organ failure and death after polytrauma.

Journal of Pharmacology and Experimental Therapeutics, 1994, 271(1): 134-142 discloses the use of anti-selectin antibodies in a neutrophil-mediated inflammation, i.e. reperfusion injury. WO-A-94 12215 also refers to the use of anti-selectin antibodies in neutrophil-mediated inflammations and defines shock, cardiac surgery and multiple organ failure as being such neutrophil-mediated inflammations. They do however not disclose oder suggest use of anti-L-selectin antibodies for the production of a pharmaceutical agent for treating a patient who has suffered a polytraumatic event. Both publications deal with inflammations and especially with the treatment of desease conditions with an inflammatory component.

### BRIEF DESCRIPTION OF THE FIGURES

Fig. 1 shows the lung wet weight of experimental animals with respect to the observation time.
Fig. 2 shows the cardiovascular parameter CO (cardiac output) with respect to time for the various experimental animals.
Fig. 3 shows the cardiovascular parameter MAP (mean arterial blood pressure) with respect to time for the experimental animals.
Fig. 4 shows the BE value (arterial base excess) with respect to time for the experimental animals.
Fig. 5 shows the number of white blood cells with respect to time for the various experimental animals.

### DETAILED DESCRIPTION OF THE INVENTION

L-selectin is a known glycoprotein that is constitutively expressed by all leukocytes. Both L-selectin and its murine homologues, GP90 and Mell4, are involved in the normal recirculation of lymphocytes - each mediates the interaction between circulating lymphocytes and vascular ligands (often referred to as "addressins") on the high endothelial venules (HEVs) of lymphoid organs (L.A. Lasky, et al., Cell 69: 927-938 (1992); E.L. Berg, et al., J. Cell. Biol. 114: 343-349 (1991)). In addition to its role as a lymphocyte homing receptor, L-selectin is also involved in the adhesion of circulating leukocytes to non-lymphoid tissues, such as endothelium, during inflammation. L-selectin is shed from the leukocyte surface following leukocyte activation (T.K. Kishimoto, et al., Science 245: 1238-1241 (1989)), and this may be an important process in retaining activated leukocytes at sites of inflammation. L-selectin has an amino-terminal carbohydrate-recognition domain (CRD) that has considerable homology with C-type lectins (K. Trickhamer, J. Biol. Chem. 263 9557-9560 (1988)), followed by a single epidermal-growth-factor-like domain, complement-regulatory domains, a single transmembrane polypeptide and a carboxy-terminal cytoplasmatic domain. L-selectin interacts with its cognate ligand through the amino-terminal CRD in a calcium dependent manner.

In accordance with the invention, anti-L-selectin antibodies are preferred which modulate, and more preferably inhibit, the interaction between the CRD domain and the corresponding carbohydrate receptors on the surface of cells. Such carbohydrate receptors are described by R.B. Parekh, Tibtech 12: 339-345 (1994), incorporated by reference. These carbohydrate receptors may be phosphorylated or sulfated sugars.

As used herein, the term "humanized immunoglobulin" refers to an immunoglobulin comprising a human framework, at least one complementarity determining region (CDR) from a non-human antibody, and in which any constant region present is substantially identical to a human immunoglobulin constant region, *i*.*e*., at least about 85-90%, preferably at least 95% identical. Hence, all parts of a humanized immunoglobulin, except possibly the CDRs, are substantially identical to corresponding parts of one or more native human immunoglobulin sequences. For example, a humanized immunoglobulin would not encompass a chimeric mouse variable region/human constant region antibody. See, e.g., European Patent Application EP A 451216, incorporated by reference

The invention concerns the use of such anti-selectin antibodies to reduce MOF and mortality after polytrauma. It has surprisingly turned out that it is possible to prevent multiple organ failure when anti-L-selectin antibodies, are administered very soon after the polytrauma. This is also surprising because there are no acute symptoms at this early stage and there would therefore have been no reason to administer such a dose as a preventive measure.

It also has surprisingly turned out that anti-L-selectin antibodies in doses of 1.0 - 10 mg/kg, preferably of 2 - 4 mg/kg, administered one to five times, preferably once or twice after the polytraumatic event can advantageously be used, where the first application is given as early as possible, preferably 0.5 - 8 hours, and especially preferably, 0.5 - 4 hours after the polytraumatic event. The intervals between the individual applications are between about 6 and about 72 hours, preferably between 6 and 36 hours.

In a preferred embodiment the dose and time of the second and subsequent preventive applications is selected depending on the concentration of the anti-L-selectin antibodies in the blood and preferably in plasma or serum, which is an early determinable parameter. In this connection it is preferable that the plasma concentration of the anti-L-selectin antibody is maintained at 10 - 100 µg/ml over a time period of 7 - 10 days after the polytraumatic event. This concentration is equivalent to about a 10 - 100 fold excess over the concentration of soluble selectin in plasma. The dose and time for the second and subsequent applications are determined by determining the concentration of the anti-L-selectin antibody in blood, serum or plasma at intervals of 6 - 24 hours and immediately administering a dose which essentially corresponds to the dose of the first application when the plasma concentration falls below 10 µg/ml antibody. When the antibody concentration is between 10 and 50µg/ml, the antibody is administered at about half the concentration of the first application, and at an antibody concentration between 50 and 100 µg/ml, no further antibody is administered. In this case only the antibody concentration is monitored further.

The anti-L-selectin antibody concentration in blood, serum or plasma is determined by the usual methods, preferably by an immunological method of determination. Such methods are known to a person skilled in the art. For example the determination can be carried out by means of an ELISA test in which a labelled L-selectin specific antibody, preferably the antibody which is also used therapeutically, competes for L-selectin . In a subsequent step the amount of labelled antibody which has bound to the antigen is then determined and the concentration of the anti-selectin antibody in the sample is determined from this.

The therapeutic compositions of the invention are usually administered parenterally such as intravenously, intraarterially, intraperitoneally, subcutaneously or intramuscularly. Intravenous (i.v.) administration is preferred. The active components of the composition can be used in a liquid or solid form, preferably in a lyophilized form and be used together with a suitable diluent or carrier such as water or aqueous solutions of sodium chloride, dextrose, buffers and so forth. Other suitable pharmaceutical auxiliary substances can also be added.

Antibodies to L-selectin are known from the state of the art and are described for example in EP-A 0 386 906, WO 93/00111 and WO 94/12215 and by Kishimoto, T.K. et al., in Blood 78: 805-811 (1991) and Proc. Natl. Acad. Sci. USA 87: 2241-2248 (1990).
L-selectin is also denoted LECAM-1, Mel 14 or Lam-1 in the literature. The cloning and sequence of Lam-1 have been described in WO 93/02698. Antibodies which bind specifically to L-selectin are suitable. Humanized antibodies, especially HuDreg 200 which is described in WO 94/12215, are suitable. Other antibodies which bind to L-selectin such as HuDreg 55, (sequence: SEQ ID NO: 1 - 4), are also particularly preferred.

"Antibody" as used herein is understood as a protein that is composed of one or several polypeptide chains which are essentially encoded by antibody genes. The antibody genes code for the antigen-specific variable regions and may also code for the genes for the constant regions. Antibodies within the sense of the invention are also understood as various derivatives and fragments of antibodies such as Fv, Fab and F(ab)₂ and individual antibody chains (Houston et al., PNAS USA 85 5879-5883 (1988), Bird et al., Science 242: 423-426 (1988), Hood et al., Immunology, Benjamin N.Y., 2nd edition (1984), Hunkapiller and Hood, Nature 323 15-16 (1986)). Monoclonal antibodies and fragments thereof are preferably used and particularly preferably chimeric or humanized antibodies preferably of the IgG1 or IgG4 subtype.

The antibodies preferably contain at least two light polypeptide chains and two heavy polypeptide chains. Each of these chains contains a variable region (usually the N-terminal part of the polypeptide chain) which in turn contains a domain which binds the antigen. Heavy and light chains additionally contain a constant region of the polypeptide (usually the C-terminal part) which mediates the binding of the antibody to leukocytes (neutrophils, lymphocytes etc.). Usually the light and heavy chains are complete antibody chains which are composed of the variable region and the complete constant region. In this connection, the variable regions and the constant regions can be derived from different antibodies, for example different isotypes. For example a polypeptide which contains the variable region of a heavy chain of an anti-L-selectin antibody to the γ-1 isotype may be linked to the constant region of the heavy chain of an antibody from another class (or subclass).

Anti-L-selectin antibodies are also suitable in which one or several amino acids are substituted. In this case, amino acids are preferably substituted by other amino acids with similar characteristic features (e.g. the acidic amino acid Asp by the acidic amino acid Glu). The structural characteristics of the original sequence are not changed by such substitutions. Examples of such polypeptide structures are described in Proteins, Structures and Molecular Principles, Creighton (editor), W.H. Freeman and Company, New York (1984) ; Introduction to Protein Structure, C. Brandon and J. Tooze, Garland Publishing, New York (1981); Thornton et al., Nature 354 105 (1991). In general, antibodies which are suitable as anti-L-selectin antibodies are those which bind to L-selectin, and/or inhibit the rolling of leukocytes (e.g. neutrophils).

In addition to the humanized immunoglobulins specifically described herein, other "substantially homologous" modified immunoglobulins can be readily designed and manufactured utilizing various recombinant DNA techniques well known to those skilled in the art. Human antibodies, including, for example, the Eu or GAL antibodies, as well as other human antibodies known in the art, can be used as a source of framework sequence. These framework sequences should exhibit a high degree of sequence identity with the mouse Dreg 55 or mouse Dreg 200 variable region domains from which the CDRs were derived. The heavy and light chain variable framework regions can be derived from the same or different human antibody sequences. Indeed, the heavy and light chain framework regions can each be derived from more than one human antibody. The human antibody sequences can be the sequences of naturally occurring human antibodies or can be consensus sequences of several human antibodies. See Carter et al., WO 92/22653 (1992).

"Antibodies which are capable of binding in an equivalent manner" are understood as those antibodies which bind to the same or an overlapping epitope of a L-selectin. Epitope overlap can be determined by methods known in the art, for example with the aid of a competitive test system. A competitive binding assay may be carried out for this and the extent to which the antibody competes with, e.g., HuDreg 55 for binding to an immobilized L-selectin antigen is determined. For this, L-selectin immobilized in a suitable manner (preferably L-selectin on leukocytes) is incubated with HuDreg 55 in a labelled form and an excess of the antibody to be tested. The extent of the binding of the antibody to be tested to L-selectin is determined in comparison to HuDreg 55 by determining the bound label of the anti-leucocyte-bound label. If the labelled HuDreg 55 is displaced by at least 50 % by the antibody to be tested an epitope overlap is present. Antibodies that bind in an equivalent manner as HuDreg 55 are preferred for use in the invention.

"Antibodies which are capable of binding in an equivalent manner" can also be identified by screening for the capacity to block neutrophil-endothelial cell interaction. A simple visual assay for detecting such interaction has been described by Kishimoto et al. *(Blood*, 78:805 (1991)). Briefly, monolayers of human umbilical vein cells are stimulated with interleukin-1. Neutrophils, with or without pretreatment with the antibody under test, are added to the monolayer under defined conditions, and the number of adhering neutrophils is determined microscopically. In one method, the neutrophils are obtained from human leukocyte adhesion deficient patients. *See* Anderson et al., *Ann. Rev. Med*. 38:175 (1987). The neutrophils from such patients lack integrin receptors, whose binding to neutrophils might obscure the effects of blocking L-selectin binding.

The antibodies can be used as complete monoclonal antibodies, fragments thereof (Fv, (Fv)₂, Fab', F(ab')₂), chimeric, humanized or human antibodies. Short antibody fragments which only contain the CDR regions or parts thereof which bind specifically to L-selectin can also be used.

The production of antibodies and in particular of monoclonal antibodies and fragments thereof is familiar to a person skilled in the art and described for example in E. Harlow and D. Lane, Antibodies: A Laboratory Manual, Cold Spring Harbor Press (1988), Bessler et al., Immunobiol. 170: 239-244 (1985), Jung et al., "Angewandte Chemie" 97: 883 (1985), Cianfiglia et al., Hybridoma Vol. 2: 451-457 (1993).

Anti-L-selectin antibodies that can be used according to the invention can also be produced by recombinant means. Such processes are described in Sambrook et al., Molecular Cloning: A Laboratory Manual, 2nd edition (1989), Cold Spring Harbor, New York, Berger and Kimmel, Methods in Enzymology, Vol. 152, Guide to Molecular Cloning Techniques (1987), Academic Press Inc., San Diego CA, which are incorporated by reference. Such recombinant antibodies can be produced either in eukaryotic or prokaryotic cells by processes known to the art. Mammalian cells, especially lymphocytic cell lines, are preferably used as host cells. Chimeric, humanized or human antibodies are preferably produced by recombinant methodalogue. Regions can be selected for the non-antigen binding regions of the antibodies which are for example described in E.A. Kabat et al., Sequences of Proteins of Immunological Interest (1987), National Institute of Health, Bethesda MD. The production of recombinant anti-L-selectin antibodies of humanized and human antibodies is described in WO 94/12215, which is hereby incorporated by reference. A particularly preferred, humanized anti-L-selectin antibody is HuDreg 55, which may be constructed in the same manner as HuDreg 200 described therein, and comprises two light chains having the sequence SEQ ID NO: 2 and two heavy chains having the sequence SEQ ID NO: 4.

Preferred humanized immunoglobulins are those which bind to L-selectinwith a binding affinity of at least 1 x 10⁷ M⁻¹ in standard binding conditions (*e.g.*, phosphate-buffered saline with 2 percent fetal bovine serum at 25°C). Examples of such humanized immunoglobulins are HuDreg 55 and HuDreg 200. More preferred are humanized antibodies, which preferably bind, in standard binding conditions, to human L-selectinwith an affinity of at least 1 x 10⁸ M⁻¹, and more preferably, with an affinity of at least 1 x 10⁹ M⁻¹, and advantageously with an affinity of at least 1 x 10¹⁰ M⁻¹ or more. Usually, the binding affinity of a humanized immunoglobulin is within a factor of 3-10 of the mouse immunoglobulin from which it was derived. For example, the affinity of the mouse Dreg 200 antibody is about 10⁸ M⁻¹ and that of mouse Dreg 55 is about 10⁹ M⁻¹.

The following examples, sequence protocols, publications and figures further elucidate the invention. The described processes are to be understood as examples of illustration, not of limitation, which also after modifications, describe the subject matter of the invention.

### EXAMPLE 1 - Use Of Anti-L-Selectin Antibody To Reduce Post-Trauma Organ Failure

The protective action of a humanized antibody against L-selectin (anti-L-selectin) in reducing post-traumatic organ failure such as that which typically occurs after injury in patients with severe polytrauma is demonstrated. Humanized anti-L-selectin antibody (HuDreg 55) is used as the antibody. It also reacts with baboon L-selectin. This mouse form of this antibody is described by Kishimoto PNAS, USA 87 (1990) 2244-2248. The humanized sequence is shown in SEQ ID NO: 1 - 4.

The HuDreg 55 and HuDreg 200 antibodies react with L-selectin on human leukocytes; however, only HuDreg 55 reacts with L-selectin of baboon leukocytes. Therefore HuDreg 55 was used. Since HuDreg 55 and HuDreg 200 bind in the same concentration range to human leukocytes (e.g. in FACS analysis), the effects of HuDreg 55 on baboons is presumptively equivalent to the effect of HuDreg 200.

As a model, severe tissue damage with associated hypovolemia (= loss of liquid and blood towards the inside and/or outside) was induced in baboons. The pure blood loss with subsequent shock (hemorrhagic shock) is less relevant for the lung damage (Pretorius et al., J. Trauma 1987; 27: 1344 - 1353; Schlag et al., page 384-402, in Schlag, Red1: Pathophysiology of Shock, Sepsis, and Organ Failure, Springer Verlag, Berlin, 1993). This is in agreement with clinical experience which shows that lung complications only occur very rarely in pure hemorrhagic shock (Schlag et al., 1993 see above).

In order to determine the frequency and severity of post-traumatic lung failure it was necessary to observe the animals (named: SELEC 971, SELEC 979 (treated); and Co 968, Co 969, Co 970 (control)) over several days; however, for ethical reasons, it was not possible to induce bone fractures in conscious animals and leave them untreated for several days so that in this subchronic model the tissue trauma is simulated. The activation of the complement system appears to be the earliest trigger for the activation of cellular systems and plays a key role in the rapid occurrence of a non-bacterial inflammatory reaction of the body (Schlag et al., 1993, supra). Therefore, in the model, complement was activated by cobra venom factor. The mortality for multiple organ failure after severe polytrauma is given as 15 - 30 % in the relevant literature. In the present animal model the severity of the polytrauma was increased to the extent that the mortality is at least twice as high and occured earlier than in humans. Therefore the observation period was limited to three days.

Adult baboons with a body weight (BW) between 18 and 22 kg were admitted to the study after three months quarantine. The fasted animals were sedated with ketamine (6-8 mg/kg), subsequently intubated and attached to a CPAP respirator (continuous positive airway pressure) (inspiratory O₂ concentration of 25 ± 2%). The anesthesia was maintained with 1-3 mg/kg/h pentobarbital. The animals breath spontaneously. A Swan Ganz catheter was pushed forward into the pulmonary artery via the right femoral vein. A catheter for withdrawing blood and measuring blood pressure was tied intc the right arm artery. A large lumen catheter is introduced into the femoral artery for the temporary collection of blood. A catheter for infusions, medication and blood collection was introduced into the left arm vein. The bladder was catheterized for the measurement of urine production. The Swan Ganz catheter and the arterial catheter were left for three days. For fluid balance the animals received 5 ml/kg/h Ringer solution (electrolyte solution for parenteral liquid substitution) during the anaesthetic phase. The blood temperature of the animals was kept at ≥ 37°C with the aid of an infrared lamp. Blood gas analyses were carried out (pO₂, pCO₂, pH, BE, HCO₃-) and hemodynamic parameters were determined (MAP, RAP, PAP, CO, HR). Lung function was determined by means of the respiratory rate (RR) and end expiratory CO₂. Blood samples were collected repeatedly in order to measure the number of white blood cells (WBCs). Cobra venom factor was administered at a dose of 10 U/kg per i.v. at the beginning of the retransfusion and administered again at a dose of 5 U/kg 1 hour after beginning the retransfusion of the blood. The blood withdrawal for triggering the hypovolemia was regulated such that the MAP (mean arterial pressure) came to lie between 40 and 50 mm Hg and the CO (cardiac output) is reduced by 50 to 70%. Approximately 50 ml/kg blood were usually withdrawn for this and stored until retransfusion. The deficient circulation was maintained for two to three hours and was controlled in such a way that the base excess was no more than -5 to -7 mEq. At the end of this shock phase retransfusion of the previously collected blood was begun. This phase lasted 4 hours.

The retransfusion was complemented by an additional administration of Ringer solution. Humanized antibody HuDreg 55 or the corresponding volume of saline solution as a placebo was administered intravenously 15 minutes after the start of retransfusion. Anti-L-selectin antibody was administered at a dose of 2 mg/kg. At the end of retransfusion the animals were awakened from anaesthesia and were returned to their cages for observation. At times 24 h, 48 h and 72 h a low level of anesthesia was again induced and the measuring parameters were registered and blood was withdrawn. If the animals had not died before the end of the three day observation period, they were then sacrificed and autopsied. The main terminal points of the study were mortality, survival period and organ damage, for example, to the lung.

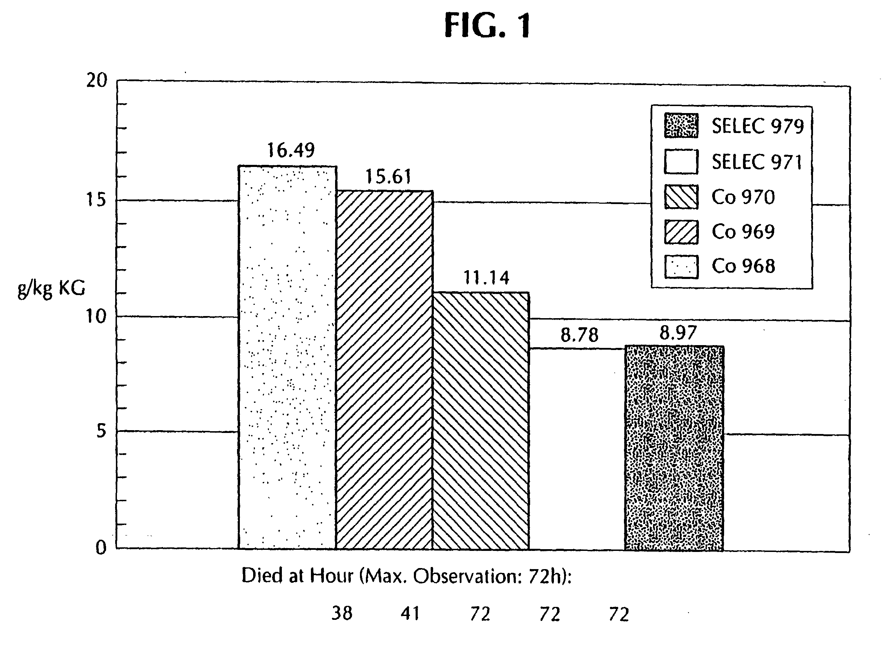

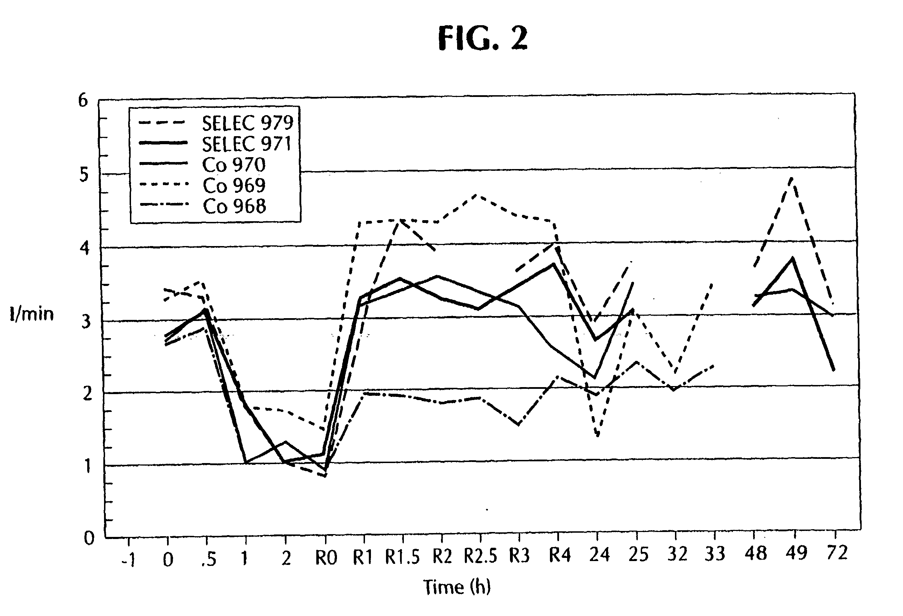

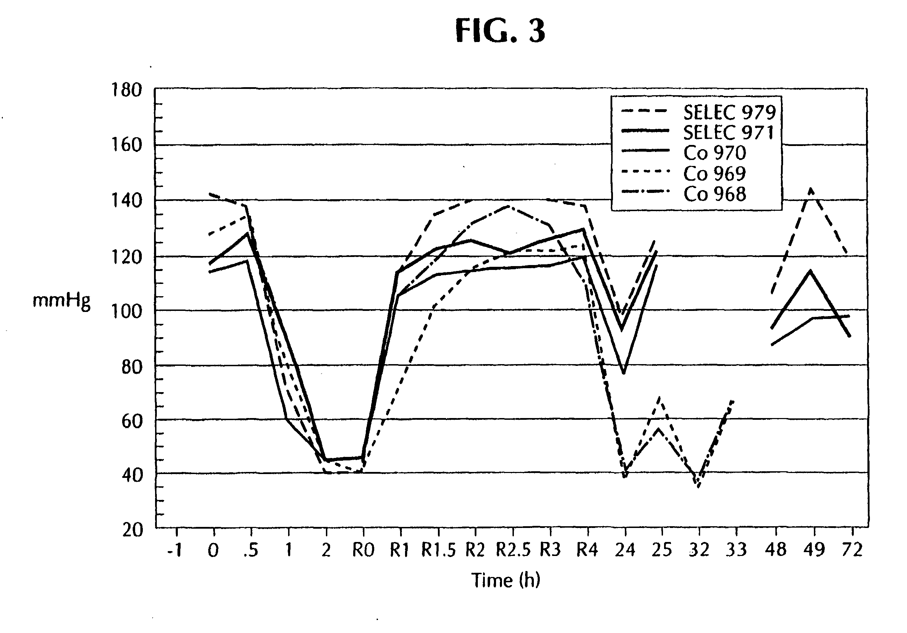

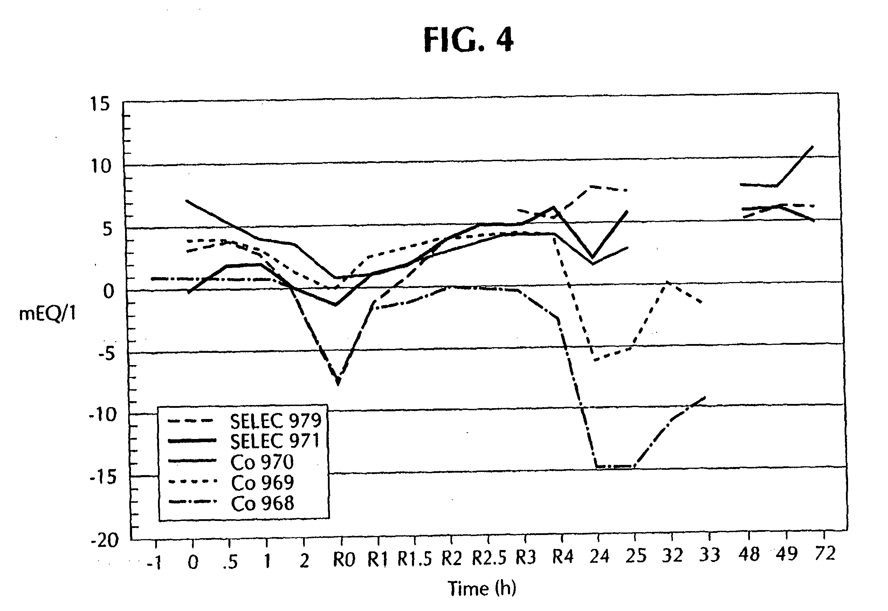

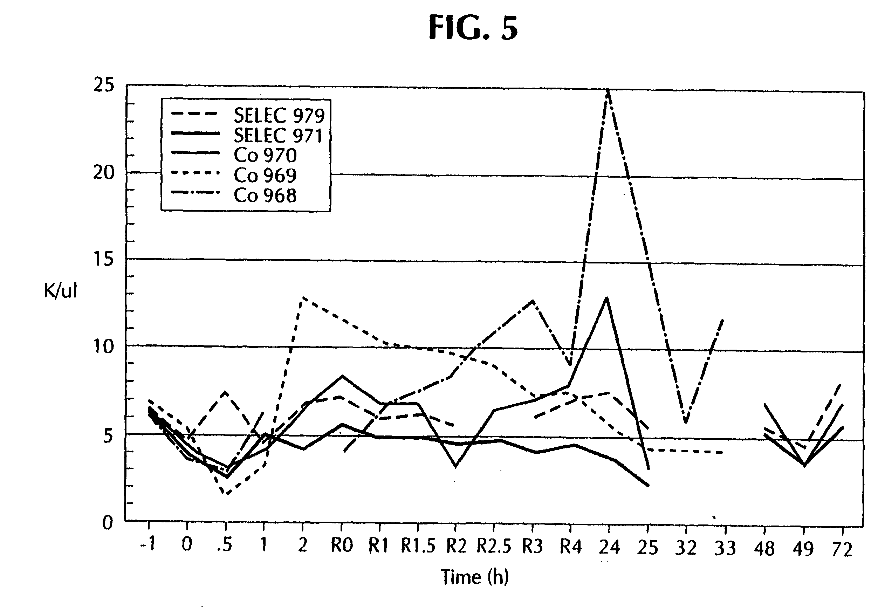

In the first experiment, three control animals were treated with placebo solution and two with the HuDreg 55 humanized antibody. Of the three control animals, two died before the end of the three day observation period at 38 h and 41 h whereas both anti-L-selectin treated animals survived. The lung wet weight, as an expression of organ damage, was almost normal in the antibody treated animals (normal values 7-8 g/kg BW) whereas it had increased considerably in all three placebo-treated control animals (Fig. 1). This is due to infiltration of fluid after the permeability disorder. The cardiovascular parameters CO₂ and MAP (Fig. 2 and 3) are better at 24 hours in the surviving animals than in the control animals. The dying control animals also have a negative arterial base excess (BE) indicating a disturbed acid-base balance (Fig. 4). The leucocytosis (increase in white blood cells) observed in the control animals is absent in the antibody animals (Fig. 5).

### EXAMPLE 2 - Use Of Anti-L-Selectin Antibody To Reduce Post-Traumatic Mortality

The experiments reported in Example 1, supra, were continued and expanded to include 28 baboons which were randomly assigned to one of two experimental groups conducted as described in Example 1. The baboons received either 2 mg/kg i.v. of anti-L-Selectin antibody or the appropriate placebo volume-dose as control 15 minutes after initiation of reperfusion after the ischemia period. The main endpoints for statistical analysis of the study were mortality at the end of the 3-day observation period and survival time. Fisher's exact test was used for mortality analysis and the log-rank-test was used for survival time analysis. One-sided p-values (reduction of mortality or prolongation of survival time by active treatment) are reported. The null hypothesis was rejected only when the probability (p) of the calculated statistic was p<0.05.

Anti-L-selectin antibody reduced (p<0.05) mortality from 10 out of 14 (=71%) baboons in the control group to 3 out of 14 (=21%) baboons in the active treatment group at a level of statistical significance. In addition, survival time in the anti-L-Selectin group was prolonged to 64.4 h, whereas animals in the control group died earlier (p<0.05), on an average at 42.1 h. This difference was statistically significant.

The table summarizes the results:

| | Mortality | Survival time (h) |
|---|---|---|
| Anti-L-Selectin Antibody | 3/14* | 64.4±4.7⁺ |
| Placebo-control | 10/14 | 42.4±5.7 |

| | | |
|---|---|---|
| Mean ± standard error of mean; | | |
| n = 14 per group; | | |
| *, p < 0.05 by Fisher's exact test; | | |
| ⁺, p<0.05 by log-rank-test. Observation period was 72 h. | | |

These data show that early treatment of baboons suffering from ischemia-reperfusion injuries due to hemorrhgaic-traumatic shock with administration of anti-L-Selectin significantly prolongs survival time and reduces mortality as compared to placebo-control.

## Claims

1. A method for the production of a pharmaceutical agent for treating a patient who has suffered a polytraumatic event, **characterized by** incorporating a therapeutically effective amount of an anti-L-selectin antibody into a pharmaceutically acceptable carrier.

2. The method of claim 1, **characterized in that** the anti-L-selectin antibody is a humanized antibody.

3. The method of claim 2, **characterized in that** the humanized antibody is anti-L-selectin antibody HuDreg 55 or HuDreg 200.

4. The method according to one of the preceeding claims, **characterized in that** for administration 1-5 times after the polytraumatic event an amount of anti-L-selectin antibody ranging from 1,0 - 10 mg/kg body weight of patient is incorporated into the pharmaceutical acceptable carrier.

5. The method of at least one of the preceeding claims, **characterized in that** the anti-L-selectin antibody is to be administered within a time periode of up to 8 hours after the polytraumatic event.

6. Method of any one of the preceeding claims, **characterized in that** the dose of anti-L-selectin antibody incorporated into the pharmaceutical acceptable carrier is determined by the concentration of the anti-L-selectin antibody in the serum or plasma of a patient at intervals of 6 - 24 hours after administration of the previous dose wherein when
(a) the concentration of said anti-L-selectin antibody is less than 10 µg/ml of said patient's serum or plasma, then a dose at least as high as the previous dose is incorporated or when
(b) the concentration of said anti-L-selectin antibody is between 10 µg/ml and 50 µg/ml of said patient's serum or plasma, then a dose which is half that of the previous dose is incorporated.

## Patentansprüche

1. Verfahren zur Herstellung eines Arzneimittels zur Behandlung eines Patienten, der ein polytraumatisches Ereignis erlitten hat, **gekennzeichnet durch** Einbringen einer therapeutisch wirksamen Menge eines Anti-L-Selektin-Antikörpers in einen pharmazeutisch akzeptablen Träger.

2. Verfahren nach Anspruch 1, **dadurch gekennzeichnet, dass** der Anti-L-Selektin-Antikörper ein humanisierter Antikörper ist.

3. Verfahren nach Anspruch 2, **dadurch gekennzeichnet, dass** der humanisierte Antikörper der Anti-L-Selektin-Antikörper HuDreg 55 oder HuDreg 200 ist.

4. Verfahren nach einem der vorhergehenden Ansprüche, **dadurch gekennzeichnet, dass** für eine Verabreichung 1 bis 5 mal nach dem polytraumatischen Ereignis eine Menge eines Anti-L-Selektin-Antikörpers im Bereich von 1,0 bis 10 mg/kg Körpergewicht des Patienten in den pharmazeutisch akzeptablen Träger eingebracht wird.

5. Verfahren nach einem der vorhergehenden Ansprüche, **dadurch gekennzeichnet, dass** der Anti-L-Selektin-Antikörper innerhalb eines Zeitraums von bis zu 8 Stunden nach dem polytraumatischen Ereignis verabreicht wird.

6. Verfahren nach einem der vorhergehenden Ansprüche, **dadurch gekennzeichnet, dass** die Dosis des Anti-L-Selektin-Antikörpers, die in den pharmazeutisch akzeptablen Träger eingebracht wird, bestimmt wird durch die Konzentration des Anti-L-Selektin-Antikörpers im Serum oder Plasma eines Patienten in Abständen von 6 bis 24 Stunden nach der Verabreichung der vorhergehenden Dosis, wobei
(a) bei einer Konzentration des Anti-L-Selektin-Antikörpers im Serum oder Plasma des Patienten von weniger als 10 µg/ml eine Dosis eingebracht wird, die mindestens so hoch ist wie die vorherige Dosis, oder
(b) bei einer Konzentration des Anti-L-Selektin-Antikörpers im Serum oder Plasma des Patienten zwischen 10 µg/ml und 50 µg/ml eine Dosis eingebracht wird, welche die Hälfte der vorherigen Dosis beträgt.

## Revendications

1. Procédé de production d'un agent pharmaceutique pour traiter un patient qui a souffert d'un événement polytraumatique, **caractérisé par** l'incorporation d'une quantité thérapeutiquement efficace d'un anticorps anti-L-sélectine dans un support pharmaceutiquement acceptable.

2. Procédé selon la revendication 1 **caractérisé en ce que** l'anticorps anti-L-sélectine est un anticorps humanisé.

3. Procédé selon la revendication 2 **caractérisé en ce que** l'anticorps humanisé est l'anticorps anti-L-sélectine HuDreg 55 ou HuDreg 200.

4. Procédé selon l'une des revendications précédentes **caractérisé en ce que**, pour l'administration 1-5 fois après l'événement polytraumatique, une quantité d'anticorps anti-L-sélectine allant de 1,0 - 10 mg/kg de poids corporel du patient est incorporée dans le support pharmaceutiquement acceptable.

5. Procédé selon au moins l'une des revendications précédentes **caractérisé en ce que** l'anticorps anti-L-sélectine doit être administré dans une période pouvant atteindre 8 heures après l'événement polytraumatique.

6. Procédé selon l'une quelconque des revendications précédentes **caractérisé en ce que** la dose d'anticorps anti-L-sélectine incorporée dans le support pharmaceutiquement acceptable est déterminée par la concentration de l'anticorps anti-L-sélectine dans le sérum ou le plasma d'un patient à des intervalles de 6 - 24 heures après l'administration de la dose précédente, où, quand
(a) la concentration dudit anticorps anti-L-sélectine est inférieure à 10 µg/ml dudit sérum ou plasma du patient, une dose au moins aussi élevée que la dose précédente est incorporée, ou quand
(b) la concentration dudit anticorps anti-L-sélectine est située entre 10 µg/ml et 50 µg/ml dudit sérum ou plasma du patient, une dose qui est la moitié de la dose précédente est incorporée.
